Europäisches Patentamt

European Patent Office

Office européen des brevets

(11) Publication number: **0 140 557**
**A2**

# (12) EUROPEAN PATENT APPLICATION

(21) Application number: **84306242.3**

(22) Date of filing: **12.09.84**

(51) Int. Cl.⁴: **A 61 B 19/00**
**A 61 B 17/04**

(30) Priority: **12.09.83 US 531024**

(43) Date of publication of application:
**08.05.85 Bulletin 85/19**

(84) Designated Contracting States:
**CH DE FR GB LI SE**

(71) Applicant: **Blackwood, Edward Lee**
**1716 Beatty Road**
**Bozeman Montana 59715(US)**

(71) Applicant: **Kollmar, Robert B.**
**2404 Westridge Drive**
**Bozeman Montana 59715(US)**

(72) Inventor: **Blackwood, Edward Lee**
**1716 Beatty Road**
**Bozeman Montana 59715(US)**

(72) Inventor: **Kollmar, Robert B.**
**2404 Westridge Drive**
**Bozeman Montana 59715(US)**

(74) Representative: **Coleman, Stanley et al,**
**MATHYS & SQUIRE 10 Fleet Street**
**London EC4Y 1AY(GB)**

(54) **Surgical instrument.**

(57) A surgical instrument for use in suturing tissue. This instrument includes two guide tubes joined longitudinally along a side of each guide tube. A push rod is positioned inside each guide tube and the push rods are joined together at one end thereof. A double armed suture having a straight needle at each end of the single suture is used in conjunction with this instrument. The needles are loaded in the guide tubes and the push rods used to plunge the needles with a suture attached through the wound to be repaired.

EP 0 140 557 A2

./...

Croydon Printing Company Ltd.

FIG. 6

-1-

## SURGICAL INSTRUMENT

### BACKGROUND OF INVENTION

The present invention relates to a surgical instrument for stitching tissue and has particular application in the stitching of a torn meniscus in a human knee joint.

Various types of suture instruments are known in the art, including single needle instruments such as shown in U. S. Patent No. 919,138 to Drake et al; U. S. Patent No. 2,833,284 to Springer and U. S. Patent No. 3,763,860 to Clarke. A surgical tool having a spiral shaped needle is shown in U. S. Patent No. 4,204,541 to Kapitanov.

None of the devices shown in the prior art can be used effectively in stitching a torn meniscus where a "U" shaped loop needs to be formed across the tear with the suture before tying the suture ends and the space in which this "U" shaped loop is formed is of constricted dimensions.

### SUMMARY OF INVENTION

This invention pertains to a surgical instrument for suturing tissue and is ideally adapted to form a "U" shaped loop with a double armed suture having a straight needle at each end of a single suture. The instrument includes two guide tubes joined longitudinally along a side of each guide tube. A push rod is positioned inside each guide tube and the push rods are joined together at one end thereof.  The length of the push rods is selected to that upon full insertion of the two push rods into the respective guide tubes the ends of the push rods are adjacent the forward ends of the two guide tubes which are opposite the rearward ends into which the push rods were inserted.

To load this instrument with a suture, the user pulls back the push rods from the forward ends of the guide tubes and takes a double armed suture having a straight needle at each end of the suture and inserts each needle into one of the guide tubes at the forward end thereof with the end of the needle attached to the suture being inserted first. The two guide tubes, at the forward end thereof, are each provided with a slit from the forward end extending rearwardly in a longitudinal direction for a distance which is the approximate length of the needle being used.  The suture extending from each needle is then drawn through a corresponding slit and the "U" loop then formed is moved toward the rearward ends of the two guide tubes.

The user next positions the forward end of the instrument adjacent the tear or wound to be sutured.  The push rods are then forced against the two needles to push the needles through the tear and then through the skin of the patient.  Next the ends of the suture threaded through the needles are removed from the needles and tied together to complete the suturing process.

This instrument has particular application in suturing a torn meniscus in a knee joint where the two needles are forced through the meniscus on one side of the tear, inside the knee of the patient, across the tear, through the meniscus on the other side of the tear and outside the skin of the patient.  Since cross-sectional dimensions of this instrument are small, the forward end can be accurately positioned in the constricted space such as where a meniscus is to be repaired. A suture of this meniscus can be done with little surgical preparation of the patient and can be effectively accomplished in a short period of time.

This instrument fills a genuine need for suture instruments which can suture tissue from within the patient's body. With the double needle, the process is fairly simple and eliminates many problems which arise when a single needle suture instrument is used.

## BRIEF DESCRIPTION OF THE DRAWINGS

In order that the invention may be clearly understood and readily carried into effect, a preferred embodiment will now be described, by way of example only, with reference to the accompanying drawings wherein:

Fig. 1 is an elevational view of the guide tubes in accordance with the invention as described herein;

Fig. 2 is a top view of the guide tubes portion shown in Fig. 1;

Fig. 3 is a left side view of the guide tubes portion shown in Fig. 1;

Fig. 4 is a right side view of the guide tubes portion shown in Fig. 1;

Fig. 5 is an elevational view of the push rods portion of the invention described herein;

Fig. 6 is a perspective view of the push rods inserted in the guide tubes according to the present invention;

Fig. 7 is a perspective view of the double armed suture to be used with the present invention; and

Fig. 8 is a view showing the operation of the instrument according to the present invention with respect to a tear to be sutured and further shows a suture inserted with the apparatus according to the present invention.

## DESCRIPTION OF A PREFERRED EMBODIMENT   **0140557**

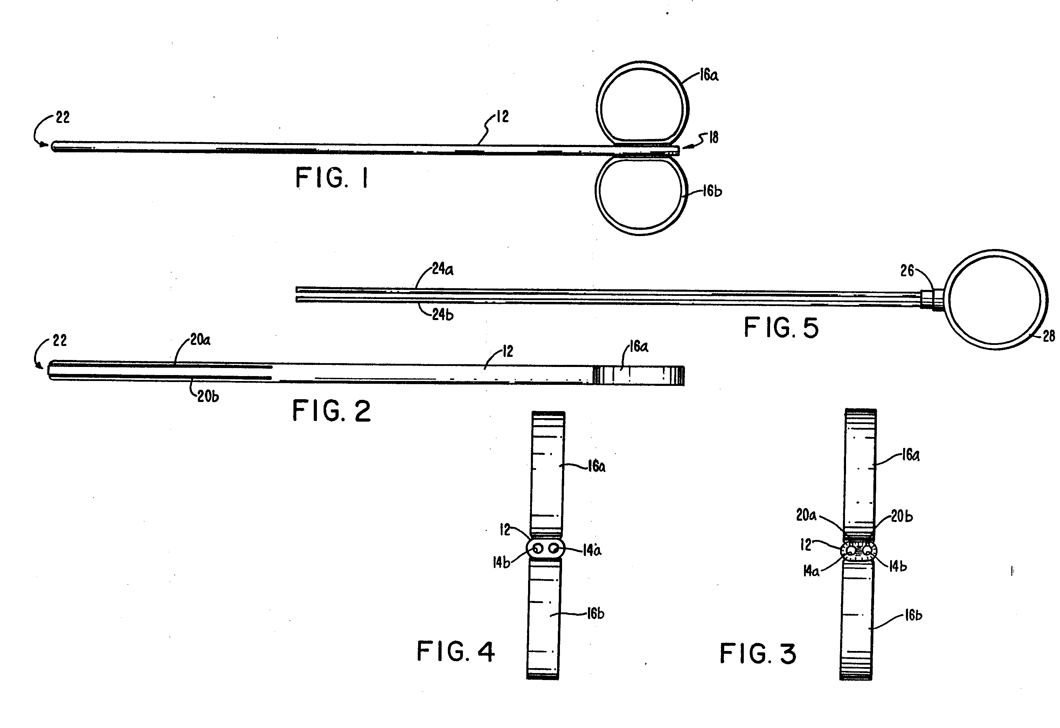

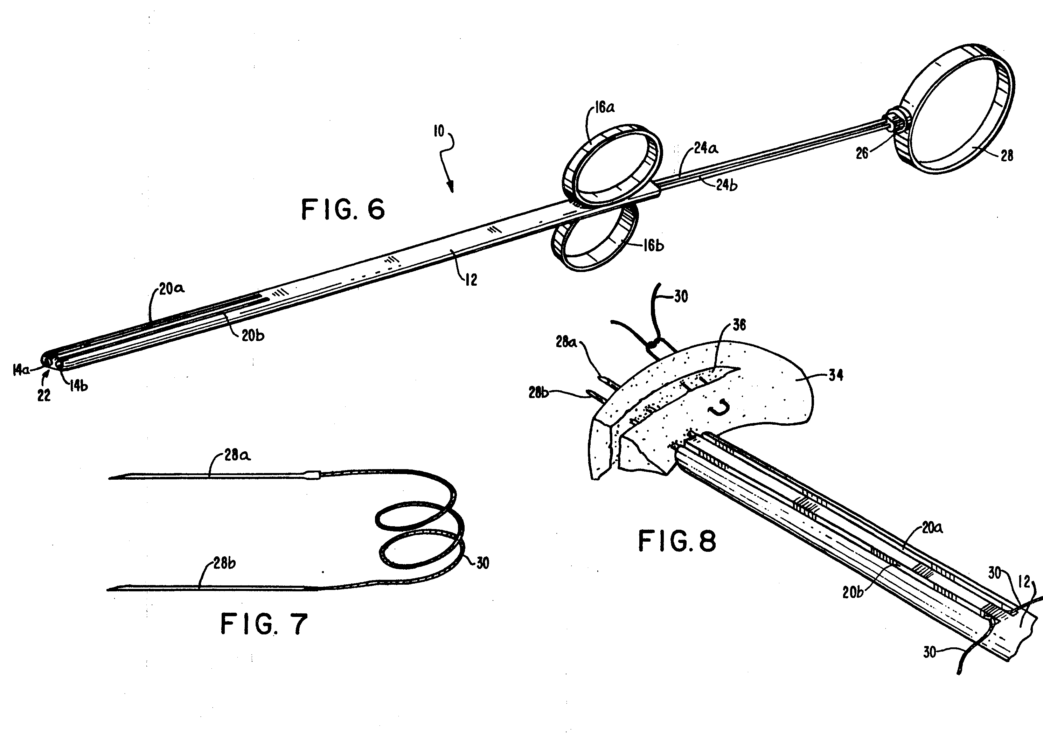

The surgical instrument 10 according to the present invention is shown in Fig. 6. This instrument includes a casing 12 having two guide tubes 14a and 14b formed therein extending the length of casing 12. Two finger grips 16a and 16b are attached to the rearward end 18 of casing 12 as shown in Fig. 1. The casing 12 has longitudinal slits 20a and 20b as shown in Fig. 2 extending from the forward end 22 of casing 12 longitudinally in the rearward direction. These slits 20a and 20b are sized to extend into the guide tubes 14a and 14b respectively as shown in Fig. 3.

The instrument 10 further includes two push rods 24a and 24b as shown in Figs. 5 and 6. These push rods are joined together at one end with a member 26 shown in Fig. 5. The member 26 is mounted by a swivel (not shown) to thumb ring 28. The push rods 24a and 24b each have a cross sectional diameter sized to slidably fit in guide tubes 14a and 14b, respectively, and each are sized in length such that upon full insertion of the push rods 24a and 24b in guide tubes 14a and 14b with the member 26 abutting the rearward end 18 of the casing 12 the forward ends of push rods 24a and 24b are approximately aligned with the forward end 22 of casing 12.

In Fig. 7 is shown a conventional double armed suture having needles 28a and 28b and suture 30. The needles 28a and 28b are crimped onto suture 30 in a conventional manner. The view of needle 28a shows an elevational view of the crimp and the view of needle 28b shows a top view of this crimp.

In using instrument 10 the push rods 24a and 24b are drawn to the right as shown in Fig. 6 with a user's thumb inserted in thumb ring 28 and two of the user's fingers inserted in finger grips 16a and 16b. The needles 28a and 28b are then inserted in guide tubes 14a and 14b, respectively, with the ends attached to suture 30 inserted first.

**0140557**

The suture 30 extending from each needle 28a and 28b is then lifted through slits 20a and 20b and the "U" loop of the suture formed between the needles is moved towards the rearward end 18 of casing 12. The instrument 10 is now ready to suture a tear or other rupture of tissue.

The following description of how the instrument 10 is used relates to the use of this apparatus in suturing a torn meniscus of a human knee joint by way of example only. It should be understood and the inventor contemplates that this instrument has broader application than simply the suturing of such a meniscus.

The user after loading the instrument 10 with the needles 28a and 28b and the suture 30 places the forward end 22 of the casing 12 in abutting relation to the meniscus 34 to be sutured as shown in Fig. 8. The meniscus 34 as shown in Fig. 8 has a tear 36 to be sutured. The thumb of the user which is inserted in thumb ring 28 is then used to plunge the push rods 24a and 24b forward causing the needles 28a and 28b to be driven through the meniscus 34 across the tear 36 to a position where the needles extend beyond the skin (not shown) of the patient. It is then an easy matter to withdraw the instrument 10 from the patient; pull the needles 28a and 28b with a grasping device (not shown) fully through the skin of the patient; cut off the needles 28a and 28b from the suture 30 and tie the ends of the suture 30 together to complete the suture. (A completed suture is also shown in Fig. 8)

The process may be repeated as many times as it is desirable to insert the necessary number of sutures to repair the torn tissue. Further, by wrapping the "U" loop end of suture 30 around the thumb of the user when the instrument 10 is loaded

- 6 -    **0140557**

with the needles and suture, the needles may be drawn back into casing 12 by simply extending the distance between the thumb and the fingers holding the instrument. This is an advantage when the user is properly positioning the instrument 10 prior to driving the needles into the meniscus.

While the fundamental novel features of the invention have been shown and described, it should be understood that various substitutions, modifications and variations may be made by those skilled in the art without departing from the spirit or scope of the invention. Accordingly, all such modifications and variations are included in the scope of the invention as defined by the following claims.

**0140557**

Claims

1. A surgical instrument for suturing tissue with a double armed suture having a needle attached to each end of the suture comprising:

a casing having at least two guide tubes formed therein;

the guide tubes each having a cross-sectional diameter sufficient to accept a needle, attached to the suture, when positioned longitudinally within the guide tube;

a push rod for each of the guide tubes with each push rod being dimensioned to slidably fit within the guide tube; and

means for driving the push rods against the needles when they are positioned within the guide tubes such that the needles are caused to move outwardly and away from the guide tubes.

2. The surgical instrument according to claim 1 wherein the casing further includes a longitudinal slit for each guide tube which slits extend longitudinally parallel to the guide tubes from a forward end of the casing where the needles are positioned in a rearward direction and wherein each slit pierces through the casing to the respective guide tube and through which slits the suture may be drawn when the needles are positioned within the guide tubes.

3. The surgical instrument according to claim 1 wherein finger grips are mounted to the casing and a thumb ring is mounted with a swivel to the ends of the push rods opposite the ends which push against the needles.

4. A surgical instrument for use in placing a single mattress stitch using a double armed suture comprising:

a casing;

at least two guide tubes formed within the casing, each of the guide tubes having a cross-sectional diameter sufficient to accept a needle, attached to the suture, when positioned longitudinally within the guide tube;

means for slidably moving the needles in a direction outwardly and away from the guide tubes; and

means for permitting the length of suture between the needles to follow the needles when the needles are moved to a position entirely outside the casing.

FIG. 1

FIG. 2

FIG. 5

FIG. 4

FIG. 3

0140557

FIG. 6

FIG. 7

FIG. 8